# EUROPEAN PATENT APPLICATION

(11) **EP 4 834 911 A1**
(43) Date of publication of application: **04.11.2026**
(21) Application number: 24913498.2
(22) Date of filing: 20.12.2024
(51) Int. Cl.: C07H 3/06, C07H 1/06

(54) **KESTOSE CRYSTAL**

(30) Priority: 26.12.2023 KR 20230191634
(71) Applicant: Samyang Corporation, Jongno-gu Seoul 03129 (KR)
(72) Inventor: RYU, Kyunghun, Seongnam-si, Gyeonggi-do 13340 (KR); KIM, Dohyun, Seongnam-si, Gyeonggi-do 13504 (KR); PARK, Jiwon, Seongnam-si, Gyeonggi-do 13544 (KR); HAN, Jungsook, Anyang-si, Gyeonggi-do 14062 (KR)
(74) Representative: Vossius & Partner Patentanwälte Rechtsanwälte mbB
(86) International application number: PCT/KR2024/020898
(87) International publication number: WO 2025/143705

(57) **Abstract**

The present application relates to a kestose crystal.

## Description

### [TECHNICAL FIELD]

The present application relates to a kestose crystal.

### [BACKGROUND ART]

Kestose is a type of fructooligosaccharides, and has been confirmed to have an enhancing effect on immunoglobulin A (IgA) antibody and an inhibiting effect on immunoglobulin E (IgE) antibody production, an activating effect on the proliferation of intestinal bifidobacteria, and an improving effect on atopic dermatitis in infants, and therefore, in order to utilize its utility as an allergy suppressing composition, an allergy suppressing food, and allergy suppressing agent, it is industrially useful to produce kestose efficiently.

However, kestose powder is amorphous and highly hygroscopic, so it easily becomes sticky and has low flowability, and is sensitive to ambient humidity, causing it to easily harden like candy during storage, making it very inconvenient to handle when used in products. Thus, a technology is needed to manufacture kestose in the form of crystalline particles with low hygroscopicity and improved flowability, which are improved in handling and use like sucrose.

### [DISCLOSURE]

### [TECHNICAL PROBLEM]

One embodiment of the present application is to provide a kestose crystal having a specific diffraction angle pattern.

Another embodiment of the present application is to provide a sweetener comprising a kestose crystal having a specific diffraction angle pattern.

Another embodiment of the present application is to provide a fructooligosaccharide comprising the kestose crystal according to one embodiment of the present application.

Another embodiment of the present application is to provide a method for preparing a kestose crystal having a specific diffraction angle pattern, comprising a step of adding seed crystals with a span of 0.5 or more and 2.5 or less to a kestose solution; and a step of obtaining a kestose crystal by cooling the kestose solution or vacuum concentrating the kestose solution.

### [TECHNICAL SOLUTION]

One embodiment of the present application relates to a kestose crystal, having an X-ray powder diffraction pattern comprising characteristic peaks at positions of 2θ diffraction angles of 15.63°, 12.90°, and 18.85° in X-ray powder diffraction (XRD) analysis.

Another embodiment of the present application relates to a sweetener comprising the kestose crystal according to one embodiment of the present application.

Another embodiment of the present application relates to a fructooligosaccharide comprising the kestose crystal according to one embodiment of the present application.

Another embodiment of the present application relates to a method for preparing the kestose crystal according to one embodiment of the present application, comprising a step of adding seed crystals with a span of 0.5 or more and 2.5 or less to a kestose solution; and a step of obtaining a kestose crystal by cooling the kestose solution or vacuum concentrating the kestose solution.

Hereinafter, the present application will be described in more detail.

The kestose crystal according to one embodiment of the present application may have an X-ray powder diffraction pattern comprising peaks at positions of 2θ diffraction angles of 15.63°, 12.90° and 18.85° in X-ray powder diffraction (XRD) analysis.

The kestose crystal according to one embodiment of the present application may have an X-ray powder diffraction pattern, comprising three highest relative intensity peaks at positions of 2θ diffraction angles of 15.63°, 12.90° and 18.85° in X-ray powder diffraction (XRD) analysis. In addition, the kestose crystal may have an X-ray powder diffraction pattern, comprising the second highest relative intensity peak at a position of 2θ diffraction angles of 12.90°, or 15.63° in X-ray powder diffraction (XRD) analysis. Furthermore, the kestose crystal may have an X-ray powder diffraction pattern, comprising the third highest relative intensity peak at a position of 2θ diffraction angles of 18.85°, or 15.63° in X-ray powder diffraction (XRD) analysis.

For example, the kestose crystal may have an X-ray powder diffraction pattern, comprising peaks at positions of 2θ diffraction angles of 15.63°, 12.90°, 18.85° and 24.27°; 2θ diffraction angles of 15.63°, 12.90°, 18.85° and 20.90°; or 2θ diffraction angles of 15.63°, 12.90°, 18.85° and 22.36° in X-ray powder diffraction (XRD) analysis.

As one embodiment, the kestose crystal may have an X-ray powder diffraction pattern, comprising peaks at positions of 2θ diffraction angles of 15.63°, 12.90°, 18.85°, 20.90° and 24.27° in X-ray powder diffraction (XRD) analysis.

As one embodiment, the kestose crystal may have an X-ray powder diffraction pattern, comprising peaks at positions of 2θ diffraction angles of 15.63°, 12.90°, 18.85°, 24.27° and 22.36° in X-ray powder diffraction (XRD) analysis.

The peak may be a peak having a relative intensity of 40% or more, 45% or more, 50% or more, 55% or more, 60% or more, 65% or more, 70% or more, 75% or more, 80% or more, 85% or more, 90% or more, or 95% or more. The relative intensity of the peak represents as a relative numerical percentage of an intensity of each peak, based on 100% of the intensity of the peak with the maximum intensity.

A certain number of peaks in order of highest relative intensity, or peaks with a relative intensity above a certain value, may be main peaks that determine a crystalline form.

For example, the main peaks may be the 1 to 5, 1 to 4, 1 to 3, or 1 to 2 peaks with the highest relative intensities.

For example, the main peaks may be peaks with relative intensities of 40% or more, 45% or more, 50% or more, 55% or more, 60% or more, 65% or more, 70% or more, 75% or more, 80% or more, 85% or more, 90% or more, or 95% or more.

As one embodiment, the kestose crystal may have an X-ray powder diffraction pattern comprising characteristic peaks at positions of 2θ diffraction angles of 15.63°, 12.90° and 18.85°; 18.85°, 12.90° and 15.63°; or 12.90°, 15.63° and 18.85°, in order of relative intensity from high to low in X-ray powder diffraction (XRD) analysis.

As one embodiment, the kestose crystal may have an X-ray powder diffraction pattern comprising characteristic peaks at positions of 2θ diffraction angles of 15.63°, 12.90°, 18.85° and 20.90°; 18.85°, 12.90°, 15.63° and 24.27°; or 12.90°, 15.63°, 18.85° and 24.27°, in order of relative intensity from high to low in X-ray powder diffraction (XRD) analysis.

As one embodiment, the kestose crystal may have an X-ray powder diffraction pattern comprising characteristic peaks at positions of 2θ diffraction angles of 15.63°, 12.90°, 18.85°, 20.90°, and 24.27°; 18.85°, 12.90°, 15.63°, 24.27° and 22.36°; or 12.90°, 15.63°, 18.85°, 24.27°, and 20.90°, in order of relative intensity from high to low in X-ray powder diffraction (XRD) analysis.

As one embodiment, the kestose crystal may have an X-ray powder diffraction pattern in which a peak with the highest relative intensity is positioned at a 2θ diffraction angle of 12.90°, 15.63°or 18.85° in X-ray powder diffraction (XRD) analysis.

When a 2θ diffraction angle is expressed as a numerical value in the present description, the 2θ diffraction angle may refer to a designated numerical value ±0.5°, ±0.4°, ±0.3°, ±0.2°, or ±0.1°.

The D[4,3] (volume mean diameter) of the kestose crystal according to one embodiment of the present application may be 170 µm or more, 180 µm or more, 190 µm or more, 200 µm or more, 210 µm or more, 220 µm or more, 230 µm or more, 240 µm or more, 250 µm or more, 260 µm or more, 270 µm or more, 280 µm or more, 290 µm or more, 300 µm or more, 310 µm or more, 320 µm or more, 330 µm or more, 340 µm or more, 350 µm or more, 360 µm or more, 370 µm or more, or 380 µm or more. The upper limit of the D[4,3] (volume mean diameter) may be for example, 1,000 µm or less, 900 µm or less, 800 µm or less, 700 µm or less, 600 µm or less, 500 µm or less, or 400 µm or less.

The mean specific surface area of the kestose crystal according to one embodiment of the present application may be 0.1 m²/g or less, 0.09 m²/g or less, 0.08 m²/g or less, 0.07 m²/g or less, 0.06 m²/g or less, 0.05 m²/g or less, 0.04 m²/g or less, 0.035 m²/g or less, 0.03 m²/g or less, or 0.025 m²/g or less. The lower limit of the mean specific surface area may be for example, 0.001 m²/g or more, 0.005 m²/g or more, or 0.01 m²/g or more.

The span of the kestose crystal according to one embodiment of the present application may be 0.5 or more to 2.2 or less, 0.5 or more to 2.1 or less, 0.5 or more to 2 or less, 0.5 or more to 1.9 or less, 0.5 or more to 1.8 or less, 0.5 or more to 1.7 or less, 0.5 or more to 1.6 or less, 0.5 or more to 1.5 or less, 0.5 or more to 1.4 or less, 0.5 or more to 1.3 or less, 0.5 or more to 1.25 or less, 0.5 or more to 1.2 or less, 0.5 or more to 1.1 or less, 0.6 or more to 2.2 or less, 0.6 or more to 2.1 or less, 0.6 or more to 2 or less, 0.6 or more to 1.9 or less, 0.6 or more to 1.8 or less, 0.6 or more to 1.7 or less, 0.6 or more to 1.6 or less, 0.6 or more to 1.5 or less, 0.6 or more to 1.4 or less, 0.6 or more to 1.3 or less, 0.6 or more to 1.25 or less, 0.6 or more to 1.2 or less, 0.6 or more to 1.1 or less, 0.7 or more to 2.2 or less, 0.7 or more to 2.1 or less, 0.7 or more to 2 or less, 0.7 or more to 1.9 or less, 0.7 or more to 1.8 or less, 0.7 or more to 1.7 or less, 0.7 or more to 1.6 or less, 0.7 or more to 1.5 or less, 0.7 or more to 1.4 or less, 0.7 or more to 1.3 or less, 0.7 or more to 1.25 or less, 0.7 or more to 1.2 or less, 0.7 or more to 1.1 or less, 0.8 or more to 2.2 or less, 0.8 or more to 2.1 or less, 0.8 or more to 2 or less, 0.8 or more to 1.9 or less, 0.8 or more to 1.8 or less, 0.8 or more to 1.7 or less, 0.8 or more to 1.6 or less, 0.8 or more to 1.5 or less, 0.8 or more to 1.4 or less, 0.8 or more to 1.3 or less, 0.8 or more to 1.25 or less, 0.8 or more to 1.2 or less, 0.8 or more to 1.1 or less, 1±0.5, 1±0.4, 1±0.3, 1±0.2, or 1±0.15.

The span is a value that represents particle size distribution, and is an index that represents a difference between the largest particle and the smallest particle in the particle size distribution. It means that farther the span is from 1, the greater the difference in particles size, and the closer the span is to 1, the more uniformly particle size are distributed. The span may be calculated by the following Equation 1: [Equation 1] $Span = \left({D}_{90}-{D}_{10}\right) / {D}_{50}$

In the Equation 1, D₉₀, D₅₀ and D₁₀ mean particle sizes of cumulative 90%, 50% and 10%, respectively, from a small particle in the cumulative particle size distribution.

The ratio of the major diameter to the minor diameter of the kestose crystal according to one embodiment of the present application may be 1 to 8, 1 to 7, 1 to 6, 1 to 5.5, 1 to 5, 1 to 4, 1 to 3, 1 to 2, or 1 to 1.5.

The melting temperature (Tm) of the kestose crystal according to one embodiment of the present application may be 180°C or more, 190°C or more, 195°C or more, 200°C or more, or 205°C or more. The upper limit of the melting temperature may be for example, 220°C or less, 215°C or less, or 210°C.

The melting enthalpy of the kestose crystal according to one embodiment of the present application may be 80 J/g or more, 85 J/g or more, 90 J/g or more, 95 J/g or more, 100 J/g or more, 105 J/g or more, 110 J/g or more, or 115 J/g or more. The upper limit of the melting enthalpy may be for example, 130 J/g or less, 125 J/g or less, or 120 J/g.

The kestose crystal according to one embodiment of the present application may comprise nistose by less than 1.4wt%, 1.3wt% or less, 1.2wt% or less, 1.1wt% or less, 1wt% or less, 0.9wt% or less, 0.8wt% or less, 0.7wt% or less, 0.6wt% or less, 0.5wt% or less, 0.4wt% or less, 0.3wt% or less, 0.25wt% or less, or 0.2wt% or less. The lower limit of the nistose content may be for example, more than 0wt%, 0.001wt% or more, 0.01wt%, or 0.1wt% or more.

The electrical conductivity of the solution of the kestose crystal according to one embodiment of the present application may be 2.5 µS/cm or less, 2 µS/cm or less, 1.5 µS/cm or less, 1.4 µS/cm or less, 1.3 µS/cm or less, 1.2 µS/cm or less, 1.1 µS/cm or less, 1 µS/cm or less, 0.9 µS/cm or less, 0.8 µS/cm or less, 0.7 µS/cm or less, or 0.6 µS/cm or less. The lower limit of the electrical conductivity may be for example, more than 0 µS/cm, more than 0.001 µS/cm, more than 0.01 µS/cm, or more than 0.1 µS/cm.

The brightness (L value) of the kestose crystal according to one embodiment of the present application may be 97.5 or less, 97 or less, 96 or less, 95 or less, 94.5 or less, 94 or less, 93.9 or less, 93.5 or less, 93 or less, or 92.5 or less. The lower limit of the brightness may be for example, 50 or more, 60 or more, 70 or more, 80 or more, or 90 or more.

The yellowness (b value) of the kestose crystal according to one embodiment of the present application may be 0.6 or less, 0.5 or less, 0.45 or less, 0.4 or less, 0.35 or less, 0.3 or less, 0.25 or less, 0.2 or less, 0.19 or less, 0.18 or less, 0.17 or less, 0.16 or less, or 0.15 or less. The lower limit of the yellowness may be for example, 0.001 or more, 0.01 or more, or 0.1 or more.

The whiteness of the kestose crystal according to one embodiment of the present application may be 95 or less, 90 or less, 85 or less, 84 or less, or 83 or less. The lower limit of the whiteness may be for example, 50 or more, 60 or more, 70 or more, or 80 or more.

The angle of repose of the kestose crystal according to one embodiment of the present application may be 47° or less, 46° or less, 45° or less, 44° or less, 43° or less, 42° or less, 41° or less, 40° or less, 39° or less, or 38.5° or less. The lower limit of the angle of repose may be for example, 20° or more, 25° or more, 30° or more, or 35° or more.

The hardness after applying a surface pressure of the same weight to the kestose crystal according to one embodiment of the present application for 2 hours under conditions of a temperature of 45°C and a relative humidity of 85% may be 1,400g or less, 1,300g or less, 1,200g or less, 1,100g or less, 1,000g or less, 950g or less, or 900g or less. The lower limit of the hardness may be for example, 500g or more, 600g or more, 700g or more, or 800g or more.

The increase hardness rate after applying a surface pressure of the same weight to the kestose crystal according to one embodiment of the present application for 2 hours under conditions of a temperature of 45°C and a relative humidity of 85% may be 120% or less, 110% or less, 100% or less, 95% or less, 90% or less, 80% or less, 70% or less, 65% or less, 60% or less, 55% or less, or 50% or less.

Another embodiment of the present application relates to a sweetener comprising the kestose crystal according to one embodiment of the present application.

Another embodiment of the present application relates to a fructooligosaccharide comprising the kestose crystal according to one embodiment of the present application.

Another embodiment of the present application relates to a method for preparing a kestose crystal, comprising a step of adding seed crystals to a raw kestose solution; and a step of obtaining a kestose crystal by cooling the raw kestose solution, or evaporatively concentrating the raw kestose solution.

In the present Example, it was confirmed that the particle size and yield were improved depending on the span of the seed crystals added during preparation of a kestose crystal. Specifically, when seed crystals with a span close to 1 were used, even if the mean size of the seed crystals was the same, it was possible to prepare a kestose crystal with a large mean particle size, uniform distribution, a low impurity content, and excellent flowability, and the crystallization yield was also improved.

The seed crystals may have a span of 0.5 or more to 2.5 or less, 0.5 or more to 2.4 or less, 0.5 or more to 2.3 or less, 0.5 or more to 2.2 or less, 0.5 or more to 2.1 or less, 0.5 or more to 2 or less, 0.5 or more to 1.9 or less, 0.5 or more to 1.8 or less, 0.5 or more to 1.7 or less, 0.5 or more to 1.6 or less, 0.5 or more to 1.5 or less, 0.5 or more to 1.4 or less, 0.5 or more to 1.3 or less, 0.5 or more to 1.2 or less, 0.5 or more to 1.1 or less, 0.6 or more to 2.5 or less, 0.6 or more to 2.4 or less, 0.6 or more to 2.3 or less, 0.6 or more to 2.2 or less, 0.6 or more to 2.1 or less, 0.6 or more to 2 or less, 0.6 or more to 1.9 or less, 0.6 or more to 1.8 or less, 0.6 or more to 1.7 or less, 0.6 or more to 1.6 or less, 0.6 or more to 1.5 or less, 0.6 or more to 1.4 or less, 0.6 or more to 1.3 or less, 0.6 or more to 1.2 or less, 0.6 or more to 1.1 or less, 0.7 or more to 2.5 or less, 0.7 or more to 2.4 or less, 0.7 or more to 2.3 or less, 0.7 or more to 2.2 or less, 0.7 or more to 2.1 or less, 0.7 or more to 2 or less, 0.7 or more to 1.9 or less, 0.7 or more to 1.8 or less, 0.7 or more to 1.7 or less, 0.7 or more to 1.6 or less, 0.7 or more to 1.5 or less, 0.7 or more to 1.4 or less, 0.7 or more to 1.3 or less, 0.7 or more to 1.2 or less, 0.7 or more to 1.1 or less, 1±0.9, 1±0.8. 1±0.7, 1±0.65, 1±0.6, 1±0.5, 1±0.4, or 1±0.3. The seed crystals may have a mean particle diameter in size of 300 µm or less, 250 µm or less, 10 to 300 µm, 50 to 300 µm, 10 to 250 µm, 50 to 250 µm, 100 to 300 µm, 100 to 250 µm, 150 to 300 µm, or 150 to 250 µm. The mean particle diameter may be a D(4,3) mean particle diameter.

The crystallization yield of the method for preparation may be 40% or more, 45% or more, 50% or more, 53% or more, or 55% or more.

The solid content of the raw kestose solution may be 70wt% or more, 75wt% or more, or 80wt% or more.

The kestose content of the raw kestose solution may be 80wt% or more, 85wt% or more, or 90wt% or more based on 100wt% of the total solid content.

The seed crystals may be added by 0.01 to 1wt%, 0.05 to 1wt%, 0.1 to 1wt%, 0.01 to 0.5wt%, 0.05 to 0.5wt%, or 0.1 to 0.5wt% based on 100wt% of the solid content of the kestose solution.

The cooling may be cooling the temperature of the raw kestose solution to a temperature of 20 to 40°C, and specifically, it may be cooling the temperature of the raw kestose solution from a temperature of 50 to 70°C to a temperature of 20 to 40°C.

The evaporatively concentrate may be concentrating by further supplying the raw kestose solution at a temperature 50 to 70°C.

### [ADVANTAGEOUS EFFECTS]

The kestose crystal according to one embodiment of the present application has a specific diffraction angle pattern with enhanced flowability and improved solidification characteristics, resulting in increased convenience of use, and excellent storage stability.

### [BRIEF DESCRIPTION OF THE DRAWINGS]

FIG. 1 is an optical micrograph of the kestose crystal of Comparative Example 1 of the present application.
FIG. 2 is an optical micrograph of the kestose crystal of Example 2 of the present application.
FIG. 3 is an optical micrograph of the kestose crystal of Example 3 of the present application.
FIG. 4 is an optical micrograph of the kestose crystal of Example 4 of the present application.

### [MODE FOR INVENTION]

Hereinafter, the present application will be described in more detail by the following Examples. However, these Examples are intended to illustrate the present application only, but the scope of the present application is not limited by these Examples.

### Example 1: Preparation of raw material solution for crystallization

45kg of heated water at 55°C added to a saccharification tank, followed by the addition of 55kg of sucrose and stirred for 1 hour to 2 hours to completely dissolve sucrose crystals. After that, the pH was titrated to 6.5 to 7.0, and then, β-fructofuranosidase derived from *Aspergillus niger*, which is an enzyme for preparing high-kestose syrup, was added, and they were reacted for 24 to 48 hours according to the preparation method disclosed in Korean Patent Publication No. 10-2018-0078065. Then, enzyme deactivation was induced by heating to 80°C for 2 hours simultaneously to titrating to pH 7.6 or more using 4N NaOH in the enzyme reaction section in which 26wt% of the sugar content remained. At the time when the enzyme deactivation was completed, the decolorization/filtration, and ion purification processes were passed through, and it was concentrated to be a solid content of 40~60 wt%.

After that, by performing high-purity separation using SMB filled with Na+ type separation resin, and concentrating, a raw material solution for crystallization containing kestose of 85wt% or more in the total solid content was obtained. Specifically, as the raw material for crystallization prepared above, a raw kestose solution was obtained with a solid content of 82.5wt%, and comprising 92.7 wt% of kestose, 4.2 wt% of nistose, and 3.1 wt% of sucrose within the total solid content.

### <Kestose content and sugar composition analysis conditions>

- Analysis equipment: HPLC Agilent, 1100 Series
- Column: Shodex Asahipak NH2P-50 4E
- Injection volume: 10µl
- Flow velocity: 1ml/min
- Column temperature: 30°C
- Mobile phase: Acetonitrile 70%
- Detector: RI detector

### Example 2: Preparation of kestose crystals using cooling method (1)

A crystallization process was performed by a method of precipitating crystals by cooling the temperature slowly using the raw material solution for crystallization comprising 92.7 wt% of kestose, 4.2 wt% of nistose, and 3.1 wt% of sucrose prepared in Example 1.

Specifically, seed crystals with the D(4,3) mean particle diameter of 200 µm and the span of 1.622 were added by 0.3 wt% relative to the solid weight of the crystallization solution to the crystallization mother liquor with an initial temperature of 60°C, pH 7.0~7.5 and the solid content of 82.5 wt%. At this time, the supersaturation level was 1.01~1.15, and crystallization was performed by cooling at a constant rate of -0.5°C/hour until reaching 30°C.

The mother liquor was removed from the cooled solution by centrifugal dehydration, thereby obtaining kestose crystals primarily. The primarily obtained kestose crystals were washed with colling water, dried and then recovered kestose crystals.

The purity analysis of the prepared kestose crystals was conducted using HPLC analysis in the same method as Example 1 above, and the purity of the prepared kestose crystals was 99.0 wt%, and the crystallization yield was 45.5%.

### Example 3: Preparation of kestose crystals using cooling method (2)

A crystallization process was performed by a method of precipitating crystals by cooling the temperature slowly using the raw material solution for crystallization comprising 92.7 wt% of kestose, 4.2 wt% of nistose, and 3.1 wt% of sucrose prepared in Example 1.

Specifically, seed crystals with the D(4,3) mean particle diameter of 200 µm and the span of 0.714 were added by 0.3 wt% relative to the solid weight of the crystallization solution to the crystallization mother liquor with an initial temperature of 60°C, pH 7.0~7.5 and the solid content of 82.5 wt%. At this time, the supersaturation level was 1.01~1.15, and crystallization was performed by cooling at a constant rate of -0.5°C/hour until reaching 30°C.

The mother liquor was removed from the cooled solution by centrifugal dehydration, thereby obtaining kestose crystals primarily. The primarily obtained kestose crystals were washed with colling water, dried and then recoverd kestose crystals.

The purity analysis of the prepared kestose crystals was conducted using HPLC analysis in the same method as Example 1 above, and the purity of the prepared kestose crystals was 99.7 wt%, and the crystallization yield was 53.9%.

### Example 4: Preparation of kestose crystals using evaporative concentration method(1)

The raw material solution for crystallization with a solid content of 60wt% comprising 92.7 wt% of kestose, 4.2 wt% of nistose, and 3.1 wt% of sucrose, and pH 7.0 to 7.5 prepared in Example 1 was added to be 25% of the reactor volume at the temperature of 60°C in vacuum, and primary vacuum concentration was performed in a reactor to be the solid content of 82.5wt%.

Seed crystals with a D(4,3) mean particle diameter of 200 µm and a span of 0.714 were added at 0.3 wt% based on the solid weight at a raw material solution for crystallization with a supersaturation of 1.01 to 1.10, and slowly stirred to generate crystal nuclei. While maintaining the vacuum state, the raw material solution for crystallization prepared in Example 1 was additionally added with the same volume of the initial solution. The second concentration was carried out while maintaining a solid content of 82.5 wt% and supersaturation, allowing the crystals to grow. At this time, without lowering the temperature, the solution was added under the constant temperature conditions ranging from 50 to 70°C, and the concentration was continued until solid content of 82.5 wt% was achieved, while maintaining the supersaturation at 1.01~1.15. Since microcrystals may be also precipitated together as the crystals grow, a small amount of water was added to dissolve the microcrystals before further adding the mother liquor, thereby preventing the inhibition of the crystal particle growth caused by the microcrystals. As above, the method for adding the raw material solution and adding water was repeatedly performed 4 times in total in the same manner.

After discharging the prepared kestose crystals from the reactor, the mother liquor was removed by centrifugal dehydration, thereby obtaining kestose crystals primarily, and the primarily obtained kestose crystals were washed with cooling water and then dried and recovered.

The purity analysis of the prepared kestose crystals was conducted using HPLC analysis in the same method as Example 1 above, and the purity of the prepared kestose crystals was 99.8 wt%, and the crystallization yield was 55.7%.

### Comparative Example 1: Preparation of kestose crystals using cooling method (3)

A crystallization process was performed by a method of precipitating crystals by cooling the temperature slowly using the raw material solution for crystallization comprising 92.7 wt% of kestose, 4.2 wt% of nistose, and 3.1 wt% of sucrose prepared in Example 1.

Specifically, seed crystals with the D(4,3) mean particle diameter of 200 µm and the span of 2.531 were added by 0.3 wt% relative to the solid weight to the raw material solution for crystallization with an initial temperature of 60°C, pH 7.0~7.5 and the solid content of 82.5 wt%. At this time, the supersaturation was 1.01~1.15, and crystallization was performed by cooling at a constant rate of -0.5°C/hour until reaching 30°C. The mother liquor was removed by centrifugal dehydration, and the crystals obtained by primary crystallization were washed with colling water and then dried and recovered kestose crystals. The purity analysis of the prepared kestose crystals was conducted using HPLC analysis in the same method as Example 1 above, and the purity of the prepared kestose crystals was 98.6 wt%, and the crystallization yield was 39.4%.

### Test example 1: Analysis of particle characteristics

The mean particle size and particle size distribution of the kestose crystals prepared in Examples 2 to 4 and Comparative Example 1 were measured using a laser diffraction type particle size analyzer, and the measured specific surface area value and span value were described in Table 1.
- Particle size analyzer: Laser Diffraction particle analyzer, Mastersizer 2000 (MALVERN Panalytical Ltd.)
- Dispersion Unit: Hydro 2000 MU (wet type)
- Dispersion solvent: Isopropyl alcohol

As shown in Table 1, the kestose crystals according to one embodiment of the present application had a large size with a mean particle size of 270 µm or more and had a very low specific surface area of 0.05 m²/g or less. In addition, the kestose crystal particles according to one embodiment of the present application showed a very uniform particle form with a span in the range of 1±0.3.

**[Table 1]**

| Sample | Mean particle size (µm) (Vol. Weighted Mean D[4,3]) | Mean specific surface area value (Specific surface area, m²/g) | Span |
|---|---|---|---|
| Comparative Example 1 | 161.2 | 0.110 | 2.214 |
| Example 2 | 275.4 | 0.047 | 1.247 |
| Example 3 | 326.3 | 0.031 | 0.904 |
| Example 4 | 385.1 | 0.022 | 0.897 |

### Test Example 2: X-ray diffraction (XRD) analysis

X-ray diffraction analysis was performed on the kestose crystals obtained in Examples 2 to 4 and Comparative Example 1 according to the following analysis conditions. The top 5 peaks of the major peaks of the X-ray diffraction pattern of the kestose crystals according to one embodiment of the present application were selected in order of relative intensity (%) from high to low, and were listed in Table 2.
- Analysis instrument: D/MAX-2200 Ultima/PC
- Manufacturer: Rigaku International Corporation (Japan)
- X-ray sauce system target: sealed tube Cu
- Tube voltage: 45 kV / tube current: 200 mA
- Scan range: 5 to 90° 2θ
- Step size: 0.02°
- Scan speed: 5°/min

**[Table 2]**

| Comparative Example 1 | | Example 2 | | Example 3 | | Example 4 | |
|---|---|---|---|---|---|---|---|
| Angle 2-Theta degree | Relative Intensity % | Angle 2-Theta degree | Relative Intensity % | Angle 2-Theta degree | Relative Intensity % | Angle 2-Theta degree | Relative Intensity % |
| 18.99 | 100.0 | 15.62 | 100.0 | 18.81 | 100.0 | 12.91 | 100.0 |
| 22.23 | 58.3 | 12.88 | 98.4 | 12.90 | 87.1 | 15.65 | 90.7 |
| 12.99 | 54.3 | 18.84 | 76.1 | 15.63 | 80.4 | 18.89 | 64.9 |
| 11.10 | 46.5 | 20.88 | 62.5 | 24.27 | 58.0 | 24.28 | 59.2 |
| 9.49 | 44.2 | 24.26 | 59.9 | 22.36 | 51.0 | 20.91 | 52.8 |

As shown in Table 2, the kestose crystals according to one embodiment of the present application prepared by performing the crystallization process by adjusting the span to 2.5 or less while preparing kestose crystals, had an X-ray powder diffraction pattern comprising characteristic peaks at positions of 2θ diffraction angles of 15.63±0.5°, 12.90±0.5°, and 18.85±0.5° in the X-ray powder diffraction (XRD) analysis. Specifically, the kestose crystals according to one embodiment of the present application had an X-ray powder diffraction pattern comprising characteristic peaks at positions of 2θ diffraction angles of 15.63±0.5°, 12.90±0.5°, 18.85±0.5°, and 24.27±0.5° in the X-ray powder diffraction (XRD) analysis. The X-ray powder diffraction pattern of the kestose crystals of Example 2 and Example 4 further comprised a characteristic peak at the position of 20.90±0.5°. The X-ray powder diffraction pattern of the kestose crystals of Example 3 further comprised a characteristic peak at the position of 22.36±0.5°.

On the other hand, in case of Comparative Example 1 using seeds with a span of over 2.5 during preparing kestose crystals, characteristic peaks of the X-ray powder diffraction pattern of the kestose crystals were shown differently.

Therefore, the kestose crystals according to one embodiment of the present application prepared by adjusting the span of the seeds in the crystallization process, had a specific crystalline form. On the other hand, not only was the size of the crystals of Comparative Example 1 very small, but also the external form of the crystals was different from the crystals according to one embodiment of the present application.

### Test Example 3: Analysis of crystal form and crystal particle size

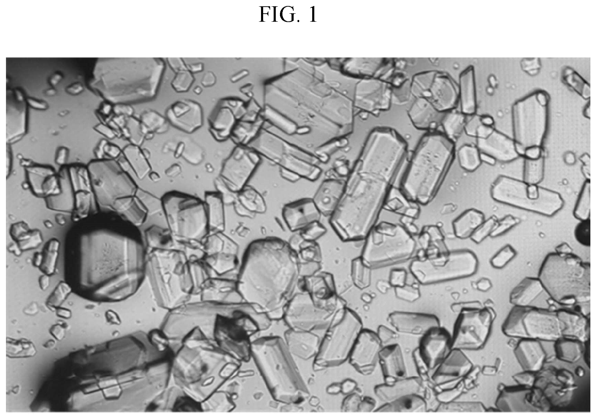

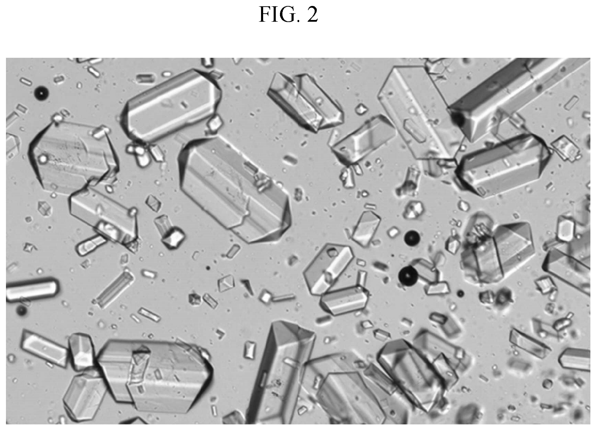

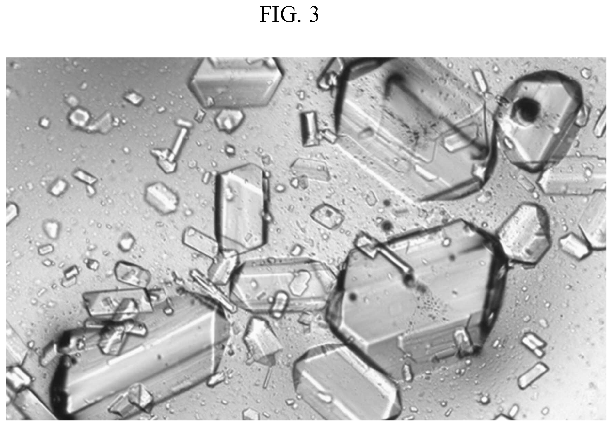

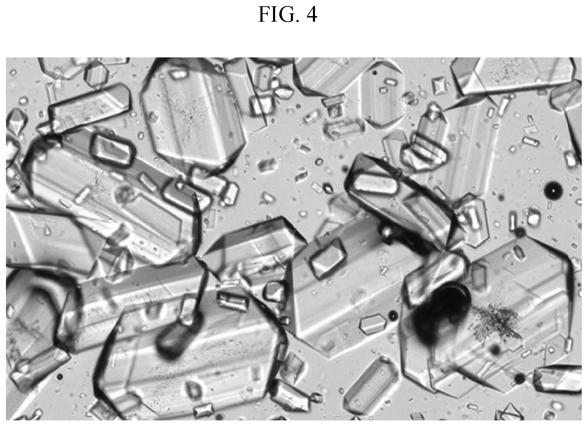

Optical micrographs of measuring the kestose crystals obtained in Examples 2 to 4 and Comparative Example 1 with magnification of X100 were shown in FIG. 1 to FIG. 4 (FIG. 1: Comparative Example 1; FIG. 2: Example 2; FIG. 3: Example 3; FIG. 4: Example 4). In addition, the major diameter (height) and minor diameter (width) were measured for 6 samples each of the kestose crystals prepared in Examples 2 to 4 and Comparative Example 1, and the particle diameter ratio (=major diameter/minor diameter) was calculated, and they were shown in Table 3. Specifically, based on 1 of the minor diameter length (µm), the ratio of the length (µm) of the major diameter was shown.

**[Table 3]**

| No. | Comparative Example 1 | Example 2 | Example 3 | Example 4 |
|---|---|---|---|---|
| #1 | 7.16 | 4.21 | 1.75 | 1.31 |
| #2 | 9.65 | 5.89 | 1.15 | 1.32 |
| #3 | 7.92 | 4.31 | 1.46 | 1.34 |
| #4 | 8.65 | 4.71 | 1.86 | 1.22 |
| #5 | 10.56 | 5.63 | 1.75 | 1.25 |
| #6 | 9.83 | 5.34 | 1.24 | 1.35 |
| Mean | 8.96 | 5.02 | 1.54 | 1.30 |

The kestose crystals have a rectangular hexahedron or a crystal structure close to it. As shown in Table 3, the kestose crystals of Example 3 and Example 4 formed tetragonal crystal structure, with each crystal plane growing uniformly to form a shape close to a square. As the crystal planes grow more uniformly, the major diameter/minor diameter ratio tends to decrease, and it is interpreted that it had a positive effect on crystal growth as the uniformity of seeds increases during preparing kestose crystals.

### Test Example 4: Differential Scanning Calorimetry (DSC) analysis

DSC analysis of the kestose crystals prepared in Examples 2 to 4 and Comparative Example 1 was performed, and specific DSC analysis conditions were as follows.
- Equipment name: DSC[differential scanning calorimetry]
- Manufacturer: Perkin Elmer
- Method: 30 to 250°C, 10°C/min temperature-raising, N2 gas purge

### (Standard method: See ASTM D 3418)

The DSC analysis results of the kestose crystals were shown in Table 4.

**[Table 4]**

| Sample | Tm(°C) | ΔH(J/g) |
|---|---|---|
| Comparative Example 1 | 176.46 | 76.7 |
| Example 2 | 198.66 | 82.1 |
| Example 3 | 202.57 | 99.7 |
| Example 4 | 205.72 | 116.0 |

As the DSC analysis results, the kestose crystals according to one embodiment of the present application had a high Tm value, and the heat capacity was also measured high. In the DSC analysis of the crystals, it can be expected that the higher the heat capacity, the kestose crystals difficult to melt easily, and the higher the heat capacity and the narrower the width of the endothermic peak, the crystals are formed uniformly and firmly. In consideration of the heat capacity and the endothermic peak enthalpy value of the kestose crystals of Example 2 to Example 4, it was confirmed that the kestose crystals according to one embodiment of the present application were formed more uniformly and firmly.

### Test Example 5: Impurity analysis

Qualitative analysis of the impurity components, such as nistose and ionic materials, remaining in the kestose crystals prepared in Examples 2 to 4 and Comparative Example 1 was performed. The nistose content (wt%) was performed using HPLC analysis in the same method as Example 1, and the ionic materials were analyzed by dissolving the kestose crystals in 10% solution and performing conductivity analysis using an electrical conductivity meter (Aquasearcher multi meter AB33M1, Ohaus). The analysis results were shown in Table 5.

**[Table 5]**

| Sample | Nistose content (wt%) | Conductivity (µS/cm) |
|---|---|---|
| Comparative Example 1 | 1.4 | 2.51 |
| Example 2 | 1.0 | 1.87 |
| Example 3 | 0.3 | 1.25 |
| Example 4 | 0.2 | 0.55 |

If the crystal particles growth is poor and they are generated as microcrystals, it becomes difficult to dehydrate and wash the mother liquor between the crystal particles. If dehydration and washing are not properly carried out, the impurity content increases. In particular, since nistose is a tetrasaccharide, it has a higher viscosity than kestose and is difficult to remove during the dehydration process. The nystose content remaining in the crystals can be used to determine whether dehydration and washing of the kestose crystals were efficiently carried out. This also significantly affects crystal purity.

As shown in Table 5, the nistose content of the kestose crystals of Example 2 to Example 4 was very low as 1wt% or less, and the conductivity numerical value was low as 2.0 µS/cm or less. This seemed to be the result of effective impurity removal during the dehydration and washing process, as the particles of the kestose crystals according to one embodiment of the present application were uniform and large.

### Test Example 6: Appearance characteristic analysis

The brightness (L value), redness (a value), yellowness (b value) and whiteness of the kestose crystals prepared in Examples 2 to 4 and Comparative Example 1 were measured using a color difference meter (Spectro color meter SA-2000, Nippon Denshoku Industries co., Ltd.) and a whiteness measuring instrument (Whiteness Tester C-130, KETT electric laboratory), and described in Table 6.

**[Table 6]**

| Sample | Color difference value (color) | | | Whiteness |
|---|---|---|---|---|
| | L* | a* | b* | Whiteness index |
| Comparative Example 1 | 97.6 | -0.04 | 0.69 | 96.4 |
| Example 2 | 94.0 | -0.03 | 0.41 | 87.3 |
| Example 3 | 93.7 | -0.03 | 0.24 | 83.2 |
| Example 4 | 92.1 | -0.02 | 0.15 | 82.6 |

The whiteness shows a higher value as it is close to white, and in general, sucrose shows a level of whiteness of 80~86. If the sucrose contains a high amount of fine particles or has a rough particle surface, the whiteness value may increase due to the difference in light scattering. The kestose crystals of Examples 2 to 4 showed a whiteness at a similar level to sucrose than the kestose crystals of Comparative Example 1, and exhibited a difference in color value, with a lower yellowness (b value) measured in the color difference value analysis. If the yellowness is high, it can be inferred that the mother liquor of the crystals is attached to the crystal particles during the dehydration and washing process after crystallization. Even if a very small amount of the mother liquor remains on the particles, it may induce browning of color or promote caking during long-term storage. The kestose crystals according to one embodiment of the present application showed a low yellowness, and this means that the formation of the crystal particles was uniformly achieved, and washing was performed smoothly during the dehydration process.

### Test Example 7: Flowability analysis of kestose crystals

The kestose crystals prepared in Examples 2 to 4 and Comparative Example 1 were prepared by 150g each, and the angle of repose was measured using automatic stirring type of angle of repose measuring equipment (manufacturer: K-one Nano., Ltd., model name: BT-200DA). The kestose crystal samples were passed through a special funnel fixed at a certain height on a complete flat reference plate of the measurement equipment, the angle of repose of the conical shape formed on the reference plate was measured, and the results were shown in Table 7. Since the slope of the accumulated crystals is measured in a state where they no longer flow on the reference plate, a smaller angle of repose indicates better flowability.

**[Table 7]**

| Sample | Angle of repose, flowability (°) |
|---|---|
| Comparative Example 1 | 47.7 |
| Example 2 | 40.0 |
| Example 3 | 38.8 |
| Example 4 | 38.1 |

As shown in Table 7, the kestose crystals of Example 2 to Example 4 improved particle flowability compared to the kestose crystals of Comparative Example 1. This can increase user convenience of kestose crystal products, and for example, convenience can be improved when kestose crystals are added or transferred to a line.

### Test Example 8: Caking hardness analysis of kestose crystals

Each of 50 g of the kestose crystal samples prepared in Examples 2 to 4 and Comparative Example 1 was sealed in the same PE zipper bag without applying external pressure and with the surface evenly. They were stored for 2 hours while maintaining the thermo-hygrostat condition of temperature 45°C and relative humidity 85%. To reproduce the caking phenomenon that may occur when crystal products were loaded and stored, moisture-absorbed samples under constant temperature and humidity conditions were sealed in PE zipper bags, and pressure was applied to the surface using a weight equal to the sample's weight to induce caking. The hardness increase rate (%) of each sample after storing for 2 hours was shown in Table 8. The caking hardness measuring condition is as follows.
- Equipment name: Texture analyzer TAXTplus (stable micro systems)
- Cylinder probe: 25mmφ Perspex
- Test speed: 2mm/sec
- Trigger force: 5g

**[Table 8]**

| Sample | Harness before storage (Force(g)) | Caking hardness after storage (Force(g)) | Hardness increase rate (%) |
|---|---|---|---|
| Comparative Example 1 | 615.8 | 1403.5 | 127.91 |
| Example 2 | 611.5 | 1181.8 | 93.26 |
| Example 3 | 602.2 | 978.7 | 62.52 |
| Example 4 | 600.5 | 892.5 | 48.63 |

As shown in Table 8, the kestose crystals of Example 4 showed the least surface caking, whereas the kestose crystals of Comparative Example 1 showed the highest caking hardness, and showed a hardness increase rate of over 120%. Such caking phenomenon are greatly influenced by the external environment during long-term storage and can be accelerated as the storage load increases, and it was confirmed that the kestose crystals according to one embodiment of the present application had improved storage stability as the degree of surface caking was alleviated even under harsh storage conditions. Therefore, the kestose crystals according to one embodiment of the present application can provide significant industrial benefit, as the storage stability is improved during packaging, storage, transportation and distribution of products.

## Claims

1. A kestose crystal, having an X-ray powder diffraction pattern comprising peaks at positions of 2θ diffraction angles of 15.63±0.5°, 12.90±0.5° and 18.85±0.5° in X-ray powder diffraction (XRD) analysis.

2. The kestose crystal according to claim 1, having an X-ray powder diffraction pattern comprising peaks at positions of 2θ diffraction angles of 15.63±0.5°, 12.90±0.5°, 18.85±0.5° and 24.27±0.5° in X-ray powder diffraction (XRD) analysis.

3. The kestose crystal according to claim 1, having an X-ray powder diffraction pattern comprising peaks at positions of 2θ diffraction angles of 15.63±0.5°, 12.90±0.5°, 18.85±0.5° and 20.90±0.5° in X-ray powder diffraction (XRD) analysis.

4. The kestose crystal according to claim 1, having an X-ray powder diffraction pattern comprising peaks at positions of 2θ diffraction angles of 15.63±0.5°, 12.90±0.5°, 18.85±0.5° and 22.36±0.5° in X-ray powder diffraction (XRD) analysis.

5. The kestose crystal according to claim 1, wherein the peak has a relative intensity of 40% or more.

6. The kestose crystal according to claim 1, wherein the D[4,3] (volume mean diameter) of the kestose crystal is 170 µm or more.

7. The kestose crystal according to claim 1, wherein the mean specific surface area of the kestose crystal is 0.1 m²/g or less.

8. The kestose crystal according to claim 1, wherein the span value of the kestose crystal is 0.5 or more to 2.2 or less.

9. The kestose crystal according to claim 1, wherein the ratio of the major diameter to the minor diameter of the kestose crystal is from 1 to 8.

10. The kestose crystal according to claim 1, wherein the melting temperature (Tm) of the kestose crystal is 180°C or more.

11. The kestose crystal according to claim 1, wherein the melting enthalpy of the kestose crystal is 80 J/g or more.

12. The kestose crystal according to claim 1, wherein the kestose crystal comprises less than 1.4 wt% of nistose.

13. The kestose crystal according to claim 1, wherein the electrical conductivity of the solution of the kestose crystal is 2.5 µS/cm or less.

14. The kestose crystal according to claim 1, wherein the brightness of the kestose crystal is 97.5 or less.

15. The kestose crystal according to claim 1, wherein the yellowness of the kestose crystal is 0.6 or less.

16. The kestose crystal according to claim 1, wherein the whiteness of the kestose crystal is 95 or less.

17. The kestose crystal according to claim 1, wherein the angle of repose of the kestose crystal is 47° or less.

18. The kestose crystal according to claim 1, wherein the hardness increase rate after applying a surface pressure of the same weight to the kestose crystal for 2 hours under conditions of a temperature of 45°C and a relative humidity of 85% is 120% or less.

19. A sweetener, comprising the kestose crystal according to any one of claims 1 to 18.

20. A fructooligosaccharide, comprising the kestose crystal according to any one of claims 1 to 18.

21. A method for preparation of the kestose crystal according to any one of claims 1 to 18, comprising:
a step of adding seed crystals with a span of 0.5 or more and 2.5 or less to a raw kestose solution; and
a step of obtaining a kestose crystal by cooling the raw kestose solution or evaporatively concentrating the raw kestose solution.

22. The method for preparation according to claim 21, wherein the seed crystals have a mean diameter of 300 µm or less.

23. The method for preparation according to claim 21, wherein the seed crystals are added by 0.01 to 1wt% based on 100wt% of the solid content of the kestose solution.

24. The method for preparation according to claim 21, wherein the crystallization yield is 40% or more.

25. The method for preparation according to claim 21, wherein the cooling is cooling the temperature of the raw kestose solution to a temperature of 20 to 40°C.

26. The method for preparation according to claim 21, wherein the evaporatively concentration is concentrating by further supplying the raw kestose solution at a temperature of 50 to 70°C.
